(19) Europäisches Patentamt
European Patent Office
Office européen des brevets

(11) **EP 2 563 208 B1**

(12) **EUROPÄISCHE PATENTSCHRIFT**

(45) Veröffentlichungstag und Bekanntmachung des Hinweises auf die Patenterteilung:
**07.01.2015   Patentblatt 2015/02**

(21) Anmeldenummer: **11733780.8**

(22) Anmeldetag: **16.04.2011**

(51) Int Cl.:
*A61B 5/00* (2006.01)     *A61B 18/20* (2006.01)

(86) Internationale Anmeldenummer:
**PCT/DE2011/000419**

(87) Internationale Veröffentlichungsnummer:
**WO 2011/134454 (03.11.2011 Gazette 2011/44)**

(54) **VORRICHTUNG MIT OCT-SYSTEM ZUR UNTERSUCHUNG UND BEHANDLUNG LEBENDEN GEWEBES UNTER ERWÄRMUNG DURCH ABSORPTION ELEKTROMAGNETISCHER STRAHLUNG**

DEVICE WITH AN OCT SYSTEM FOR EXAMINING AND TREATING LIVING TISSUE BEING HEATED BY ABSORBING ELECTROMAGNETIC RADIATION

DISPOSITIF AVEC SYSTÈME OCT (TOMOGRAPHIE PAR COHÉRENCE OPTIQUE) POUR L'EXAMEN ET LE TRAITEMENT D'UN TISSU VIVANT SOUS CHAUFFAGE PAR ABSORPTION D'UN RAYONNEMENT ÉLECTROMAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2010   DE 102010018679**

(43) Veröffentlichungstag der Anmeldung:
**06.03.2013   Patentblatt 2013/10**

(73) Patentinhaber: **Medizinisches Laserzentrum Lübeck GmbH**
**23562 Lübeck (DE)**

(72) Erfinder:
• **BIRNGRUBER, Reginald**
**23564 Lübeck (DE)**
• **BRINKMANN, Ralf**
**23562 Lübeck (DE)**
• **HÜTTMANN, Gereon**
**23564 Lübeck (DE)**
• **MÜLLER, Heike**
**22527 Hamburg (DE)**

(74) Vertreter: **Hermann, Felix**
**Boehmert & Boehmert**
**Anwaltspartnerschaft mbB**
**Patentanwälte Rechtsanwälte**
**Pettenkoferstrasse 20-22**
**80336 Munich (DE)**

(56) Entgegenhaltungen:
**WO-A2-2009/108950     DE-A1- 10 135 944**

• **VAKOC ET AL.: "Real-time microscopic visualization of tissue response to laser thermal therapy", J. BIOMED. OPT., Bd. 12, Nr. 2, 2007, Seiten 020501-1-020501-3, XP002657992, in der Anmeldung erwähnt**
• **YEH-CHAN AHN ET AL: "Quantification of a three-dimensional velocity vector using spectral-domain Doppler optical coherence tomography", OPTICS LETTERS, OSA, OPTICAL SOCIETY OF AMERICA, WASHINGTON, DC, US, Bd. 32, Nr. 11, 1. Juni 2007 (2007-06-01), Seiten 1587-1589, XP002590245, ISSN: 0146-9592, DOI: 10.1364/OL. 32.001587 [gefunden am 2007-05-18]**
• **HEIKE H. MÜLLER ET AL: "Imaging of temperature distribution and retinal tissue changes during photocoagulation by high speed OCT", PROCEEDINGS OF SPIE, Bd. 7889, 1. Januar 2011 (2011-01-01), Seiten 78890E-1-78890E-7, XP55005941, ISSN: 0277-786X, DOI: 10.1117/12.874788**

EP 2 563 208 B1

**Beschreibung**

Die Erfindung betrifft eine Vorrichtung zur Untersuchung und ggf. zur Therapie lebenden Gewebes mittels lokaler Erwärmung des Gewebes durch Absorption elektromagnetischer Strahlung, beispielsweise von Laserlicht oder Mikrowellenstrahlung. Die Erfindung betrifft ferner eine Vorrichtung zur Messung von Gewebeeigenschaften in Echtzeit. Die Erfindung betrifft überdies eine Vorrichtung zur Feedback-Dosimetriekontrolle der therapeutischen Strahlungsquelle.

Die Analyse der Expansion eines Absorbers und die damit emittierte Druckwelle nach Applikation eines kurzen Laserpulses ist in Sigrist M. W., "Laser Generation of Acoustic Waves in Liquids and Gases", Journal of Applied Physics 60(7):R83-R121, 1986 beschrieben worden.

Auf dieser Grundlage entstand die optoakustische Temperaturmessung an der Retina, wie sie in der DE 101 35 944 C2 dargestellt ist. Dabei werden durch repetitive Bestrahlung mit kurzen Laserpulsen Druckwellen erzeugt, die sich durch das lebende Auge fortpflanzen und als Drucktransienten auf der Hornhaut mit einem Ultraschallsensor (z.B. Piezo-Element) erfasst werden können. Die Amplituden der Drucktransienten lassen beispielsweise den Rückschluss auf die Temperatur am Augenhintergrund zu - zumindest im Mittel über den bestrahlten Bereich, der den Ausgangspunkt der Druckwelle bildet.

Das Verfahren der DE 101 35 944 C2 ist echtzeitfähig und somit zur Steuerung der therapeutisch wirksamen Strahlungsquelle (hier: Laser) geeignet. Es ist jedoch nicht bildgebend (d.h. es erlaubt keine ortsaufgelöste Messung), nicht kontaktlos und vor allem ist seine Anwendbarkeit auf das Regime der thermoelastischen Gewebeänderungen beschränkt. Kommt es zu persistenten Schäden, etwa zu Koagulationen oder Blasenbildung, dann sind die optoakustischen Signale grundsätzlich nicht mehr interpretierbar und zeigen typisch ein eher zufälliges Verhalten.

Um Gewebeveränderungen durch Absorption elektromagnetischer Strahlung vom Bereich der thermoelastischen Expansion bis hin zu beliebigen Gewebeschäden konsistent beobachten zu können, empfiehlt sich die Anwendung der bekannten Optischen Kohärenztomographie (Optical Coherence Tomography, OCT). Dieses Verfahren ist bildgebend, kontaktlos und heute sehr schnell durchführbar. Eine OCT-Messung (auch: A-Scan) erfolgt lokal dort, wohin man den Messlichtstrahl richtet, wobei das Messlicht in verschiedenen Tiefen der Probe zurückgestreut wird. Das wiederkehrende Messlicht wird mit einem Referenzlichtstrahl überlagert, und das gemessene Interferenzlicht erlaubt u. a. die Berechnung der Streustärkenverteilung entlang der Rückstreurichtung (gleich Einstrahlrichtung) des Messlichts. Typische Messtiefen von OCT-Systemen liegen zwischen 0,5 und 2 Millimeter. Der Messlichtstrahl kann mittels eines elektronischen Scanners seitlich abgelenkt und eine vorbestimmte Linie oder Fläche abrasternd über die Probe geführt werden (auch: B-Scan). Man kann somit das Verhalten eines wählbaren Probenschnitts oder -volumens begrenzter Tiefe observieren, insbesondere eines lebenden Gewebes. Hauptanwendungen der OCT liegen deshalb in der Ophthalmologie, der Dermatologie und in der Endoskopie.

Die WO 01/80792 A2 lehrt deshalb, dass Reflektivitäts-Tiefenprofile einer biologischen Probe mit hoher Messgeschwindigkeit mittels OCT bestimmt und bewertet werden sollen, um Gewebeveränderungen durch Lasertherapie zu erkennen. Allerdings ist der WO 01/80792 A2 kein konkreter Hinweis darauf zu entnehmen, welche Gewebeparameter für die Untersuchung der Bestrahlungseffekte oder gar für die Steuerung der Therapiestrahlung relevant sind und wie man aus den gemessenen Profilen entsprechende Interpretationen oder Maßnahmen ableiten soll. Insofern lehrt die Druckschrift nur, die Therapiebestrahlung mit OCT-Messungen zu begleiten und überlässt es ganz dem Leser, die gemessenen Daten selbst zu beurteilen.

In Anbetracht der DE 101 35 944 C2 wendet man sich zu diesem Zweck den thermomechanischen Eigenschaften des lebenden Gewebes zu.

Das Bestimmen mechanischer Eigenschaften insbesondere mittels OCT-Messungen ist Gegenstand der OCT-Elastographie (OCE). Beispielsweise aus der Arbeit von Liang et al., "Optical micro-scale mapping of dynamic biomechanical tissue properties", Vol. 16, No. 15, OPTICS EXPRESS, 11052 pp. (2008) geht ein Verfahren zur bildgebenden Bestimmung der biomechanischen Eigenschaften biologischer ex vivo Proben hervor, welches die mikroskopische, ortsaufgelöste Bewegung der Proben bei mechanischer Anregung, etwa mittels Piezoelementen, untersucht. Dabei kann die Temperatur der Probe gezielt eingestellt werden. Die OCE lässt die Probe unverändert (zerstörungsfreies Messen) und ist auf eine genaue Kontrolle der mechanischen Erregung angewiesen.

Die OCE eignet sich nur bedingt zur Messung an einem lebenden Patienten, da dann unvermeidliche Eigenbewegungen auftreten und zudem die kontrollierte mechanische Erregung nicht immer direkt am Zielgewebe vorgenommen werden kann (s. z. B. WO 2007/059292 A2, wo allerdings kein OCT, sondern ein Laser-Velocimeter an reflektierenden Flächen benutzt wird, um die Antwort auf mechanische Anregungen zu messen).

Die Arbeit von Liang et al. gibt zwei wertvolle Hinweise: zum einen soll man sich die messbaren OCT-Phasen ansehen, um die lokalen Gewebebewegungen festzustellen, und zum zweiten wird zur Phasenmessung die Verwendung eines Spectral Domain oder auch Fourier Domain (FD-) OCT empfohlen.

Beispielsweise geht aus der DE 43 09 056 A1 ein FD-OCT hervor. Dabei wird Licht aus einer kurzkohärenten Lichtquelle in der Probe in einer Ebene mit Abstand z zu einer Referenzebene (z=0) gestreut und mit zurück gestreutem Licht aus der Referenzebene überlagert. Es kommt so zu konstruktiver oder destruktiver Interferenz für einen beliebigen, festen Abstand z der Ebenen je nachdem, welche der eingestrahlten Wellenlängen λ man betrachtet. Bei Verwendung kurzkohärenten (breitbandigen) Lichts, z.B. aus einer Superlumineszenzdiode, wird das Interferenzlicht spektral zerlegt und üblicherweise auf eine Sensorzeile oder eine vergleichbare Vorrichtung abgebildet. Dies erlaubt das Messen der Verteilung I(k), k=2π/λ als räumliche Verteilung auf der Sensorzeile. Die Fouriertransformation dieser Verteilung führt auf das tiefenabhängige Streuvermögen S(z). Neben dieser als Spektralradar bezeichneten Ausführung beruht eine weitere Ausführungsform der FD-OCT, die auch Swept-Source OCT (SS-OCT) genannt wird, auf einem schnell durchstimmbaren Laser mit einem Photodetektor, der den spektralen Verlauf der Interferenz am Ausgang des Interferometers misst.

Die Arbeit von Vakoc et al. "Real-time microscopic visualization of tissue response to laser thermal therapy". J. Biomed. Opt., Vol. 12(2), p. 020501-1 (2007) stellt schließlich den nächstkommenden Stand der Technik dar. Sie demonstriert insbesondere die Messbarkeit von Gewebegeschwindigkeiten aus dem Phasensignal einer FD-OCT unter gleichzeitiger Absorption von Laserstrahlung. Die gezielte lokale Erwärmung führt zur Denaturierung von ex vivo Gewebe, und die Korrelation zwischen dem OCT-Phasensignal und der Schädigungstiefe wird anhand histologischer Befunde für verschiedene Expositionszeiten belegt. Die Messung der OCT-Phasen ist dabei in Echtzeit durchführbar.

Dies bestätigt, dass der Fortschritt einer Bearbeitung biologischen Gewebes durch Absorption elektromagnetischer Strahlung prinzipiell anhand der messbaren Phasendaten eines FD-OCT beobachtet werden kann.

Offen bleiben auch bei Vakoc et al. die Fragen nach der Behandlung von Eigenbewegungen bei der in vivo Anwendung sowie nach einer geeigneten Observablen, anhand derer thermoelastische von persistenten Gewebeexpansionen in Echtzeit - und eben nicht erst durch eine anschließende histologische Inspektion - unterschieden werden können.

Die Aufgabe der Erfindung besteht darin, eine Vorrichtung zur Untersuchung und ggf. Therapie lebenden Gewebes mittels Erwärmung durch Absorption elektromagnetischer Strahlung anzugeben, die thermomechanische Gewebeeffekte ermittelt und bei Bedarf die Strahlungsquelle auf der Basis der Messdaten steuert.

Die Aufgabe wird gelöst durch die Vorrichtung mit den Merkmalen von Anspruch 1. Die Unteransprüche geben vorteilhafte Ausgestaltungen an.

Die erfindungsgemäße Vorrichtung umfasst eine elektromagnetische Strahlungsquelle (z.B. einen Laser, eine Blitzlampe, eine Mikrowellenquelle o. ä.) sowie eine Steuereinheit zur Kontrolle der Bestrahlungsparameter der Strahlungsquelle (insbesondere Intensität, Bestrahlungsdauer, Repetitionsrate, Pulsenergie, Leuchtstärke etc.). Die Vorrichtung weist ferner wenigstens eine FD-OCT-Einrichtung auf. Die wenigstens eine FD-OCT-Einrichtung umfasst eine polychromatische Lichtquelle, optische Komponenten zur Beleuchtung des Gewebes an vorbestimmten Messorten sowie zur Rückführung des von den Messorten wiederkehrenden Lichts in eine Messeinheit. In der ebenfalls zur FD-OCT-Einrichtung zählenden Messeinheit wird das wiederkehrende Licht mit einem Referenzlichtstrahl überlagert. Die Messeinheit umfasst einen Detektor, der die spektrale Intensitätsverteilung am Ausgang des Interferometers detektiert (typisch ein linearer Zeilensensor bei Spektralradar oder eine Photodiodenanordung bei SS-OCT)

Weiterhin besitzt die erfindungsgemäße Vorrichtung eine Recheneinheit, die folgende Aufgaben ausführt:

a. Auslesen der Messdaten wenigstens eines Detektors der wenigstens einen FD-OCT-Einrichtung für einen vorbestimmten Messort auf der Probenoberfläche;
b. Berechnen der Streustärken und Phasen aus den Sensordaten entlang der wenigstens einen Einstrahlrichtung des FD-OCT-Messlichts;
c. Berechnen der lokalen Gewebegeschwindigkeiten entlang wenigstens einer Einstrahlrichtung aus den zuvor bestimmten Phasen;
d. Wiederholen der Schritte a. bis c. in vorbestimmten Zeitabständen für denselben Messort;
e. Integrieren der unter c. bestimmten lokalen Geschwindigkeiten über die Zeit durch Aufsummieren der unter c. bestimmten Daten über eine vorbestimmte Mehrzahl von Wiederholungen gemäß Schritt d.;
f. Differenzieren der unter e. berechneten Integrale nach wenigstens einer Koordinate des die Orte der gemessenen Streustärken und Phasen indizierenden Koordinatensystems;
g. Bereitstellen der nach Schritt f. berechneten Daten zur Ausgabe (z.B. auf einem Sichtgerät) und/oder zur Weiterbewertung und Entscheidung zur Ansteuerung der Kontrolleinheit der Strahlungsquelle.

Der letztgenannte Schritt g. wird weiter unten erläutert.

Die erfindungsgemäße Vorrichtung geht - in ihrer einfachsten Ausgestaltung - also über Vakoc et al. insoweit hinaus, dass sie zusätzlich mindestens die Schritte e. und f. ausführt. Wenngleich dies zunächst Verfahrensschritte sind, ist die Implementation in eine Recheneinheit unumgänglich, da die Ausführbarkeit der später erläuterten Dosimetriekontrolle voraussetzt, dass die Berechnungen binnen Sekundenbruchteilen zu erfolgen haben. Ohne Automation wäre dies nicht möglich.

Die Erfindung erschließt eine neue Ebene der Messdateninterpretation, die nachfolgend im Detail erklärt werden soll. Aus dieser neuen Interpretation ergeben sich Erweiterungsoptionen der Vorrichtung, die ebenfalls Gegenstand dieser Erfindung sind.

Die Erfindung wird auch anhand von Figuren erläutert. Dabei zeigt:

Fig. 1 ein Intensitätsbild der Streustärkenverteilung in der Netzhaut des Auges mit der RPE-Schicht, die durch eine weiße waagrechte Linie markiert ist, aufgezeichnet mit einem FD-OCT-Linienscan. Vertikale weiße Linien kennzeichnen ausgewählte Tiefenprofile;

Fig. 2 Plots verschiedener gemessener und berechneter Größen entlang der Tiefenachse für die ausgewählten Profile aus Fig. 1;

Fig. 3 eine Skizze der Vorrichtung umfassend eine FD-OCT-Einrichtung (OCT), eine elektromagnetische Strahlungsquelle (EM) zum Bestrahlen und lokalen Erwärmen einer Probe und eine Recheneinheit (CPU) sowie eine Ablenkeinrichtung für das FD-OCT-Messlicht;

Fig. 4 eine modifizierte Skizze der Vorrichtung aus Fig. 3, nunmehr ausgebildet zum Erfassen von drei linear unabhängigen Komponenten des lokalen Geschwindigkeitsfeldes in der erwärmten Probe.

Mit heutigen FD-OCT-Systemen ist ein A-Scan - die Aufzeichnung eines einzelnen Tiefenprofils für einen einzelnen Messort - innerhalb von 10 $\mu$s möglich. Die Tiefenauflösung der Messung liegt bei etwa 10 $\mu$m, und die Messtiefe eines FD-OCT beträgt typisch 500 - 1000 $\mu$m und ist auch abhängig von den Eigenschaften der Probe.

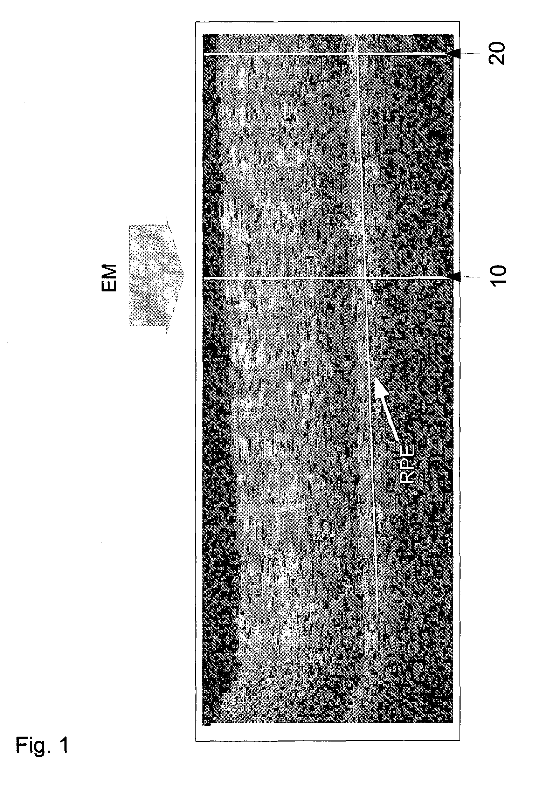

Fig. 1 zeigt ein typisches FD-OCT-Bild, das sich bei einer entlang der Oberfläche einer Retina geführten Messlichtbeleuchtung (Linien-Scan) ergibt. Dargestellt sind die messbaren Streustärken als Intensitätsplot, wobei hellere Pixel höhere Streustärken indizieren.

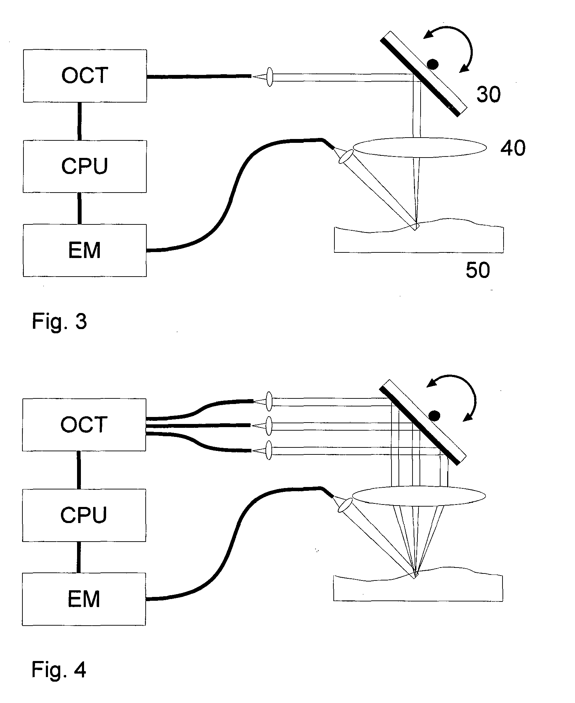

Das FD-OCT detektiert die spektrale Intensitätsverteilung des Interferenzlichts I(k,t) (dabei ist k= $2\pi/\lambda$ die Wellenzahl eines Messlichtanteils, t ist die Zeit). In Schritt b. wird diese Verteilung vorzugsweise komplex fouriertrans-formiert gemäß $\int$ I(k,t) exp(ikz) dk, und es ergibt sich die Streustärke S(z,t) als Absolutbetrag und $\varphi$(z,t) als Phase der Fourierkoeffizienten. Mit z wird hier die Ortskoordinate entlang der Einstrahl- bzw. Rückstreurichtung des Messlichts bezeichnet. Diese wird üblich senkrecht zur Gewebeoberfläche eingerichtet, doch dies ist nicht notwendig. Auch ein schräger Einfall des Messlichts kann zweckmäßig sein (s. etwa Fig. 4 und Erläuterungen dazu).

Es ist von der so genannten Doppler-OCT her bekannt, dass die Ableitung der Phase nach der Zeit ein Maß für die lokale Geschwindigkeitskomponente der Streuer in Richtung des Strahles ist, d.h. d$\varphi$/dt $\sim$ v(z,t). In der Praxis berechnet man anstelle des Differential- den Differenzenquotienten $\Delta\varphi/\Delta t$ mit einem nicht zu kleinen $\Delta t$, da es in der Messung zahlreiche Störquellen gibt, die ein statistischen Phasenrauschen nach sich ziehen. Wäre $\Delta t$ zu klein, würde dieses informationslose Rauschen numerisch überbetont, d.h. das Signal-RauschVerhältnis wäre ungünstig. Anderer-seits darf $\Delta t$ höchstens so groß sein, dass sich die optische Wegstrecke zum lokalen Streuer bei gegebener Wellenlänge (z.B. FD-OCT Weißlicht 820 $\pm$ 40 nm) und gegebener Geschwindigkeit während des Intervalls $\Delta t$ um nicht mehr als die halbe Wellenlänge (hier z.B. 410 nm) bewegt. Anderenfalls würden sich die Phasen um mehr als $\pi$ ändern, was in der Messung aber nicht erkannt werden könnte. Es käme zu entsprechenden Fehleinschätzungen der Streuergeschwin-digkeiten. Somit existieren Werte $t_{min}$ und $t_{max}$, für die $t_{min} < \Delta t < t_{max}$ gelten muss, um eine stabile Messung der Streuergeschwindigkeit am Messort aus den Phasen vornehmen zu können. Dabei hängt $t_{min}$ von der konkreten Mess-vorrichtung, insbesondere von deren Rauschverhalten, ab, während sich $t_{max}$ an der mittleren Messlichtwellenlänge und an der maximal zu erwartenden Streuergeschwindigkeit orientiert. Zur Reduktion des Messaufwandes (möglichst wenige A-Scans) wird man vorzugsweise $\Delta t$ in der Nähe von $t_{max}$ wählen.

Beispielsweise für die Messung von Gewebegeschwindigkeiten bis hin zu 100 $\mu$m/s reicht es aus, A-Scans in Zeitabständen von $\Delta t$ = 5 ms durchzuführen und auszuwerten. Sind höhere Geschwindigkeiten zu erwarten, sollte man $\Delta t$ verringern.

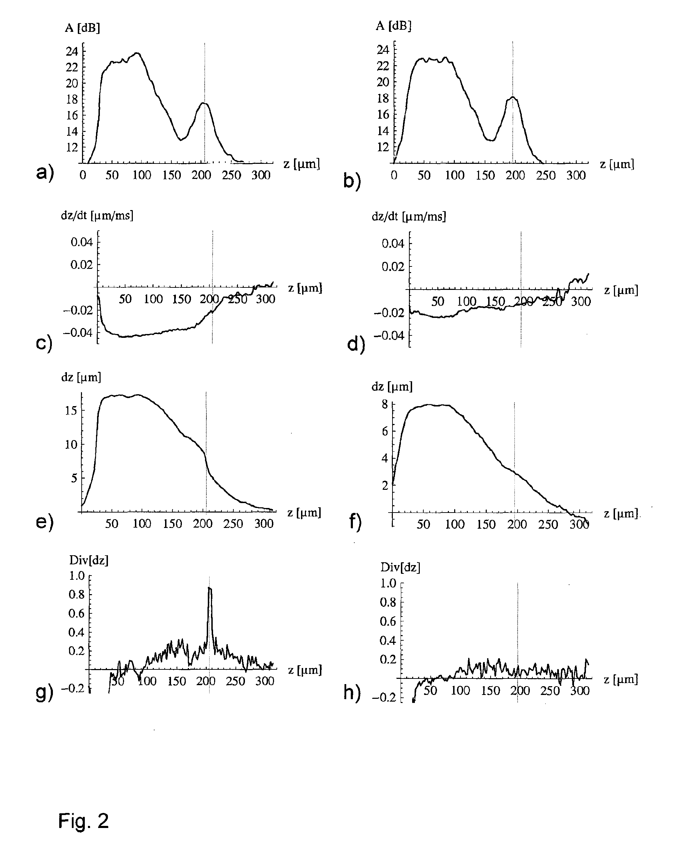

Bei der FD-OCT-Messung aus Fig. 1 werden Streustärken, Phasen und Gewebegeschwindigkeiten zu vorbestimmten Zeitschritten gemessen, während zugleich eine Laserbestrahlung stattfindet. Der bestrahlte Bereich ist mit einem breiten Pfeil (EM) gekennzeichnet. Zum Zeitpunkt des Entstehens von Fig. 1 ist bereits für etwa 500 ms eine Laserleistung von 94 mW appliziert worden. Die mit RPE gekennzeichnete weiße Linie markiert den Verlauf der RPE-Schicht, die bekanntlich einen Großteil des Laserlichts absorbiert. Zwei vertikale weiße Linien kennzeichnen ausgewählte Tiefenprofile 10 und 20, entlang derer die Messdaten genauer in Fig. 2 gezeigt werden. Dabei liegt das Tiefenprofil 10 im Zentrum der Lasereinstrahlung, hingegen das Tiefenprofil 20 außerhalb des beleuchteten Flecks.

In Fig. 2 a) und b) sind die Streustärken für die Profile 10 und 20 dargestellt. An diesen ist praktisch kein Unterschied auszumachen, d.h. es ist schwierig, zwischen bestrahltem und nicht bestrahltem Gewebe allein anhand der Streustärken zu unterscheiden.

Die Graphiken in Fig. 2 c) und d) zeigen die aus den Phasen bestimmten, instantanen Gewebegeschwindigkeiten für die Profile 10 und 20, respektive. Die Laserbestrahlung ist an den deutlich höheren Geschwindigkeiten (bis hin zu 40 µm/s in Profil 10 ablesbar. Jedoch ist auch daran nicht abzulesen, in welcher Tiefe welcher Anteil der eingestrahlten Energie absorbiert wird. Das negative Vorzeichen der Geschwindigkeit besagt, dass sich das Gewebe auf die Ebene z=0 an der Retinaoberfläche zu bewegt. Es kommt der Einstrahlrichtung also entgegen, wie man dies durch die Expansion erwarten darf.

Die instantane Gewebegeschwindigkeit - berechnet durch Differenzbildung der Phasen zwischen zwei aufeinander folgenden A-Scans - ist gewöhnlich stochastisch verrauscht, enthält zudem Anteile zusätzlicher Oszillationen, wird bei lebendem Gewebe durch Eigenbewegungen beeinflusst und lässt keinen Unterschied erkennen zwischen elastischen und persistenten Effekten.

Demgegenüber ist das Zeitintegral, das im Folgenden als Verschiebungsfeld D bezeichnet wird, ein kaum verrauschter Mittelwert, wobei die Integration zugleich als Tiefpass wirkt. Das Verschiebungsfeld D ist nichts anderes als die tatsächliche Verschiebung von Streupunkten im Gewebe infolge der Absorption elektromagnetischer Strahlung als Funktion der Zeit und der Koordinate z in Einstrahlrichtung.

D(z,t) ist im Prinzip ein Vektorfeld, doch nur die Vektorkomponente in z-Richtung ist messbar. Das Zeitintegral kann zwischen beliebigen Zeitpunkten gebildet werden. Bevorzugt wird man als Integrationsanfang eine Zeit vor oder bei Einsetzen der Absorptionsbestrahlung wählen und als Integrationsende gewöhnlich eine Zeit nach dem Abschalten der Strahlungsquelle.

In Fig. 2 e) und f) sind die z-Komponenten der Verschiebungsfelder in den Profilen 10 und 20, respektive, dargestellt. Dabei zeigt sich bereits eine steile Schulter in Fig. 2 e) in einer definierten Tiefe von etwa 200 µm unterhalb der Retinaoberfläche. Differenziert man beide Verschiebungen nach der Tiefenkoordinate z, wird der Unterschied zwischen bestrahltem und nicht bestrahltem Gewebe besonders augenfällig, wie die Graphiken Fig. 2 g) und h), respektive, zeigen. Eine senkrechte Linie markiert in allen Graphiken der Fig. 2 die Lage des Maximums aus Fig. 2 g).

Zu jedem Zeitpunkt t ist der Differenzenquotient $[D(z+\Delta z,t) - D(z,t)]/\Delta z$ die Abstandsänderung zweier Punkte, die sich ursprünglich im Abstand $\Delta z$ zueinander befanden. Mithin folgt für infinitesimales $\Delta z$, dass die Ableitung $dD/dz$ die lineare Gewebeexpansion, $\varepsilon(z,t) = dD(z,t)/dz$, angibt. Im Falle der rein thermoelastischen Expansion des Gewebes (reversible Expansion, keine Denaturierung o. ä.) lässt sich hieraus direkt auf die Temperaturerhöhung im Gewebe schließen.

$$(1) \qquad \varepsilon(z,t) = \alpha_L(T)\, \Delta T$$

Dabei ist $\alpha_L$ der - im Allgemeinen auch temperaturabhängige - lineare thermische Expansionskoeffizient und $\Delta T$ der Temperaturanstieg. Für biologische Gewebe und auch für Wasser sind Expansionskoeffizienten bekannt. Sofern nur volumetrische Expansionskoeffizienten $\alpha_V$ bekannt sind, kann man sich mit der Näherung für isotrope Medien $\alpha_L \approx \alpha_V/3$ behelfen.

Soweit die im Gewebe durch Absorption elektromagnetischer Strahlung deponierte Energie nicht ausreicht, eine Veränderung des Gewebes herbeizuführen, erlaubt die erfindungsgemäße Vorrichtung unmittelbar die Messung der ortsaufgelösten thermoelastischen Gewebeexpansion (oder -kontraktion) und mittelbar auch der tiefenaufgelösten Temperaturverteilung entlang einer Scanlinie in Einstrahlrichtung. Die so gewonnenen Daten stellen stets Momentaufnahmen zur Zeit t dar.

Vorteilhafterweise kann der Messlichtstrahl in an sich bekannter Weise über ein Gewebeareal abrasternd geführt werden. Dies ermöglicht das Durchführen einer Vielzahl von A-Scans auf einem vorbestimmten Raster der Gewebefläche (B-Scan, entlang x- und y-Koordinatenachsen). Es ist bekannt, die Messdaten aller A-Scans zusammenzufassen und so ein dreidimensionales Abbild des Gewebes zu erhalten.

Ein Ausführungsbeispiel für eine erfindungsgemäße Vorrichtung zur Durchführung von B-Scans ist in Fig. 3 dargestellt. Das polychromatische Messlicht entstammt einer Lichtquelle, die in der FD-OCT-Einrichtung (OCT) integriert ist. Es wird über eine Faser geführt, am Austrittsende kollimiert und auf einen periodisch schwenkbaren Ablenkspiegel (30) gelenkt. Dieser lenkt das Messlicht auf eine abbildende Optik (40), die es auf die Probenoberfläche (50) fokussiert. Der Fokus läuft über die Probe (50) während der Bewegung des Ablenkspiegels (30). Die FD-OCT-Einrichtung (OCT) umfasst zudem die Mittel zur Überlagerung des von der Probe zurück gestreuten Lichts mit einem Referenzlichtstrahl und zur Detektion des Interferenzlichts nach dem Stand der Technik. Die Messdaten der FD-OCT-Einrichtung (OCT) werden über eine in Fig. 3 angedeutete Datenleitung der Recheneinheit (CPU) zur Analyse und Aufzeichnung bereitgestellt. Die Recheneinheit (CPU) stellt beispielsweise die Messdaten für eine volle Periode der Bewegung des Ablenkspiegels (30) als B-Scan-Datensatz indiziert durch den Messort auf der Probe und die Zeit zusammen und speichert diesen nach der Fertigstellung. Sie berechnet Streustärken und Phasen aus den FD-OCT-Messdaten, bildet die Differenzen zwischen den Phasendaten aufeinander folgender B-Scans (zeitliches Differenzieren zur Berechnung der lokalen Geschwindigkeitskomponente), summiert diese Phasendifferenzen kumulativ auf (zeitliches Integrieren zur Berechnung der lokalen Verschiebungskomponente D(x,y,z,t)) und differenziert das jeweils aktualisierte Zeitintegral nach der Tiefenkoordinate. Die ebenfalls nach der Fertigstellung jedes B-Scans aktualisierte räumliche Ableitung beschreibt die lineare Expansion $\varepsilon$ (x,y,z,t) der Probe (50) in Einstrahlrichtung als Funktion des Messortes auf der Probenoberfläche, der Tiefe in der Probe und der Zeit.

Weiterhin ist in Fig. 3 die elektromagnetische Strahlungsquelle (EM) dargestellt, deren Licht über eine eigene Faser geführt und separat auf die Probe eingestrahlt wird. Die Strahlungsquelle (EM) umfasst hier auch die Steuereinheit zur Kontrolle der Bestrahlungsparameter, die nach dem Stand der Technik gängig in baulicher Einheit mit der eigentlichen Lichtquelle vorliegt. Es ist in Fig. 3 eine Datenverbindung zwischen der Strahlungsquelle (EM) und der Recheneinheit (CPU) vorgesehen. Über diese Datenverbindung kann die Recheneinheit (CPU) die eingestellten Bestrahlungsparameter abfragen und/oder geänderte Bestrahlungsparameter vorgeben, mithin die Strahlungsquelle (EM) ansteuern.

Für die von der Strahlungsquelle (EM) emittierte Strahlung sind kaum Einschränkungen vorgesehen. Sie muss in der Probe (50, hier: lebendes Gewebe) nur absorbiert werden. Das gesamte elektromagnetische Spektrum unterhalb der Röntgenstrahlung kommt in Betracht. Gepulste oder cw-Bestrahlung sind möglich, ebenso kann mono- oder polychromatisches Licht verwendet werden. Insbesondere könnte sogar die FD-OCT-Messlichtstrahlung selbst zur Erwärmung des Gewebes dienen, wenn eine hohe Intensität eingestrahlt wird. Normalerweise ist die Intensität des FD-OCT-Messlichts zu gering, um das Gewebe nennenswert zu beeinflussen.

Die Einstrahlung elektromagnetischer Energie zur Erwärmung des Gewebes durch Absorption erfolgt für gewöhnlich zielgerichtet und auf einen kleinen Bereich (Spot) begrenzt. Dies gilt insbesondere für die Lasertherapie. Das somit interessierende Areal ist üblich auf eine Umgebung um diesen Spot begrenzt. Die erfindungsgemäße Vorrichtung weist daher bevorzugt einen Scanner zur Ablenkung des FD-OCT-Messlichts auf, der einen Scanbereich mit dem Spot als Zentrum besitzt. Dies könnte konstruktiv auch dadurch befördert werden, dass der Applikator für die zu absorbierende Strahlung starr mit dem Scanner verbunden ist, dem das Messlicht über die Lichtleiterfasern zugeführt wird. Alternativ kann der Scanner auch wellenlängenselektiv ausgebildet sein und das Messlicht in den Strahlengang des Therapiestrahls eingespiegelt werden, so dass er nur den Wellenlängenbereich der Messlichtstrahlung ablenkt, während die therapeutisch wirksame Strahlung (z.B. Mikrowellen) unabgelenkt den Scanner passiert.

Der Scanner kann ein Flächen- oder ein Linienscanner sein. Vorzugsweise wird ein Linienscanner dabei das Zentrum des Spots überstreichen.

Ein Mapping der Gewebeexpansion in z-Richtung $\partial D(x,y,z,t)/\partial z$ auf die Temperaturverteilung im lebenden Gewebe ist möglich, solange nur thermoelastische Bewegungen stattfinden. $\partial D/\partial z$ ist eine zur Beurteilung der Gewebeexpansion ausreichende Messgröße, solange die mikromechanische Isotropie des Gewebes unterstellt werden kann. Vorteilhafterweise ist man nicht unbedingt auf die Isotropieannahme angewiesen. Da die bislang beschriebene, erfindungsgemäße Vorrichtung immer die Komponente des Verschiebungsfeldes entlang der Einstrahlrichtung misst, ist eine bevorzugte Ausgestaltung darin zu sehen, eine Mehrzahl von simultanen, nicht parallelen Messlichtstrahlen vorzusehen. Insbesondere kann man bei gleichzeitiger schräger Einstrahlung aus drei linear unabhängigen Richtungen auch drei linear unabhängige Komponenten des Verschiebungsfeldes vermessen. Fig. 4 zeigt eine Ausgestaltung der Vorrichtung, bei der als einziger Unterschied zu Fig. 3 vorgesehen ist, dass das FD-OCT-Messlicht aus drei parallel gerichteten Fasern kollimiert austritt und auf den Ablenkspiegel trifft. Dabei sollen die drei Faserenden die Eckpunkte eines Dreiecks bilden. Licht aus jeder Faser gelangt so auf einen anderen Punkt der abbildenden Optik und wird zum Messort hin gebrochen. Effektiv werden die drei Messlichtstrahlen dann aus drei linear unabhängigen Richtungen auf jeden Messort eingestrahlt, und es bedarf dabei nur eines einzelnen Scanners, um alle Messlichtstrahlen gemeinsam über die Probe zu führen. Die unterschiedlichen Einstrahlrichtungen kann man alternativ auch durch die Verwendung dreier unterschiedlich zur Probe orientierter und so fixierter, Messlicht führender Fasern realisieren, die direkt auf die Probe einstrahlen (nicht dargestellt). Diese Strahlen stets zeitgleich über dasselbe Areal auf der Probe zu führen wäre jedoch aufwendiger. Es ist dabei nicht zwingend erforderlich, drei FD-OCT-Systeme parallel zu betreiben. Vielmehr kann man in einem festen Zeittakt das Messlicht zyklisch durch jede der drei Fasern leiten. Beispielsweise kann nach jedem abgeschlossenen B-Scan eine andere Faser aktiviert werden.

Auf die Verwendung von Fasern ist man nicht angewiesen, so dass das Vorgesagte nicht einschränkend aufgefasst werden soll. Bekanntlich kann man jeden fasergeführten optischen Aufbau auch ohne Fasern realisieren. Dies ist nur gewöhnlich unpraktisch.

Bei der FD-OCT-Messung aus drei linear unabhängigen Raumrichtungen ergeben sich einige Registrierungsschwierigkeiten, auf die an dieser Stelle hingewiesen werden soll. Zum einen erfasst man alle Messdaten a priori in einem schiefwinkligen Koordinatensystem. Zum anderen erfasst man einzelne Geschwindigkeitskomponenten stets zeitgleich entlang einer dieser Koordinatenachsen. Gewöhnlich wird man infolge der Diskretisierung der Abrasterung durch die Messlicht-Scanner nur wenige Voxel des Probenvolumens haben, für die man zwei oder gar drei Komponenten des Geschwindigkeitsfeldes direkt misst. Und selbst dann werden die verschiedenen Komponenten auch noch zu verschiedenen Zeiten bestimmt. Man müsste einigen numerischen Aufwand betreiben, um das komplette Geschwindigkeitsfeld als Funktion von Ort und Zeit durch dreidimensionale Interpolation einigermaßen genau zu bestimmen. Zur Durchführung der Erfindung ist die Aufgabe glücklicherweise einfacher, da die Zeitintegration eine Interpolation entlang der Zeitachse unnötig macht. Bei der Ortsregistrierung sind Interpolationen (der Zeitintegrale, also der Verschiebungen) hingegen angebracht, und es ist zu beachten, dass nicht für alle Voxel der Probe alle drei Komponenten des Verschiebungsfeldes überhaupt vorliegen. Außerhalb eines Schnittvolumens, das von alle drei Tiefenscans durchsetzt wird, fehlen entsprechende Messdaten. Man sollte deshalb das interessierende Schnittvolumen ausreichend groß festlegen und die nicht darin befindlichen Messdaten aus der Auswertung entfernen.

Mit der Messung aller drei Komponenten des Verschiebungsfeldes kann auch für nichtisotrope Proben die Divergenz des Verschiebungsfeldes D(x,y,z,t) berechnet werden. Sie gibt die dreidimensionale Gewebeexpansion infolge der Absorption elektromagnetischer Strahlung an.

$$(2) \qquad \varepsilon(x,y,z,t) = \text{div } D(x,y,z,t) = \alpha_V(T)\, \Delta T$$

In der Physik hat die Divergenz eines Feldes die Bedeutung seiner Quellstärke. Sie ist invariant gegenüber Translationen des Feldes. Somit sind Eigenbewegungen des lebenden Gewebes insoweit aus der Messung eliminiert, wie sie das betrachtete Gewebe als Ganzes betreffen. Lediglich Expansionen, die beispielsweise auf Blutpuls in Kapillaren innerhalb des betrachteten Gewebes zurückgehen, werden immer noch erfasst. Sie zeichnen sich jedoch durch eine charakteristische Frequenz (ca. 1 Hz) aus und können als solche isoliert werden.

Das Zeitverhalten der Divergenz des Verschiebungsfeldes kann ebenfalls physikalisch interpretiert werden. Die nachfolgenden Aussagen gelten sowohl für die dreidimensionale als auch für die eingangs beschriebene eindimensionale Messung der Gewebeexpansion. Es wird dabei erläutert, wie der Schritt g. der zu Beginn genannten Aufgaben der Recheneinheit zu bewerkstelligen ist.

Wird ein absorbierendes Volumen mit elektromagnetischer Strahlung beaufschlagt, so steigt seine Temperatur zunächst linear an. Ein Teil der eingestrahlten Energie wird durch Wärmediffusion in die Umgebung transportiert, sobald ein ausreichend hoher Temperaturgradient vorliegt, der einen effektiven Wärmetransport ermöglicht. Der Anstieg der Temperatur am Absorptionsort lässt dadurch bei Fortsetzung der Einstrahlung nach. Mit Hilfe der Wärmeleitungstheorie lässt sich der zeitliche Temperaturverlauf an einem unveränderten Absorbervolumen genau vorhersagen, wenn man (lokal) das Absorptionsvermögen und die Wärmediffusionskonstante kennt. Umgekehrt lassen sich beide Werte aus dem Zeitverlauf der gemessenen Temperaturverteilung berechnen, wenn man sicherstellt, dass keine irreversiblen Änderungen (z.B. Phasenübergänge, Denaturierungen) am Gewebe auftreten.

Wird die elektromagnetische Strahlung in einer Dosis in das Gewebe eingestrahlt, die für das Auslösen irreversibler Gewebeänderungen sicher nicht ausreicht, dann ist die zu messende Gewebeexpansion $\varepsilon = \text{div } D$ als Funktion der Zeit durch Anwendung von Gleichung (2) ein direktes Maß für den erzielten, tiefen- bzw. ortsaufgelösten, dynamischen Temperaturanstieg und erlaubt über ein Wärmeleitungsmodell den Rückschluss auf das tiefen- bzw. ortsaufgelöste Absorptionsvermögen sowie die Wärmediffusionskonstante.

Mit Hilfe dieser Gewebeparameter kann die Recheneinheit der erfindungsgemäßen Vorrichtung nun eine Vorhersage des Temperaturfeldes berechnen, das sich ergeben müsste, wenn die Leistung der Therapiestrahlung um ein vorbestimmtes Inkrement erhöht wird. Sie weist dann die Steuereinheit der Strahlungsquelle an, entsprechende Einstellungen zur Realisierung dieses Inkrements vorzunehmen (z.B. Intensitätssteigerung, Erhöhung der Pulsrate eines Pulslasers oder dergleichen). Nach dem Ende des folgende Scans der FD-OCT-Einrichtung erstellt die Recheneinheit ein Update von D, $\varepsilon = \text{div } D$ und des modellierten Temperaturfeldes und vergleicht letzteres mit der Vorhersage.

Liegen die Unterschiede zwischen ermittelter und vorhergesagter Temperaturverteilung innerhalb tolerabler Grenzen (bedingt durch Messunsicherheiten), so wird auf Basis der aktuellen Messung die nächste Vorhersage erstellt und der Steuereinrichtung ein weiterer Inkrement-Befehl erteilt. Zeigen sich hingegen Unterschiede in einem Ausmaß, das sich nicht mehr durch Messunsicherheiten erklären lässt, so ist davon auszugehen, dass im Gewebe zusätzliche Expansionen oder Kontraktionen durch Gewebeänderungen stattgefunden haben, insbesondere kommen hierfür Protein-Denaturierungen in Betracht. Zudem ändert das Gewebe dann lokal seine optischen Eigenschaften, insbesondere Streu- und Absorptionsvermögen, so dass das Einsetzen solcher Umwandlungen zu deutlichen Abweichungen von der - allein auf Wärmeleitung im inerten Medium basierenden-Vorhersage führen muss.

Das Auftreten solcher Abweichungen kann zum Anlass genommen werden, die Therapiebestrahlung abzubrechen. Die Recheneinheit hat dabei nicht nur ein physikalisch-und nicht nur empirisch - begründetes Abbruchkriterium zur Verfügung, sondern überdies auch eine Momentaufnahme der Gewebeexpansion zum Zeitpunkt des Einsetzens der Gewebeänderung, die vorzugsweise sofort abgespeichert wird. Damit ist auch im Nachhinein noch eine genaue Analyse der erzielten Gewebeschäden möglich.

Für die Zwecke der Dosimetriekontrolle ist es ratsam, die therapeutische Strahlungsquelle zu Beginn der Bestrahlung auf Parameter einzustellen, die gewiss keine Schäden im Gewebe hervorrufen können. Erst wenn die Recheneinheit nach einer Startphase ausreichende Daten für eine stabile Temperaturmodellierung zur Verfügung hat, ist es sinnvoll, die therapeutisch wirksame Strahlungsdosis schrittweise zu steigern. Das vorgenannte Inkrement soll also auch ausdrücklich den Fall gleich bleibender Bestrahlungsparameter im nächsten Zeitschritt einschließen. Das Inkrement der Leistung der Therapiestrahlung ist also größer oder gleich Null.

Die Realisierung bzw. Aktivierung der Dosimetriekontrolle wie zuvor beschrieben ist optional. Die erfindungsgemäße Vorrichtung kann auch lediglich dazu verwendet werden, die zuvor genannten Gewebeparameter zu vermessen oder zu kartieren. Die Vorrichtung ist somit ein Therapie- und ein Diagnosegerät. Die elektromagnetische Strahlungsquelle, deren Strahlung im lebenden Gewebe zum Zwecke der lokalen Erwärmung absorbiert wird, ist in beiden Anwendungen erforderlich, um die Energiedeponierung präzise zu steuern. Es ist ein wichtiger Aspekt der Erfindung, dass diese Energiedeponierung direkt in das vom FD-OCT-Scan überwachte Gebiet erfolgt.

Zum Abschluss soll noch auf eine Erweiterungsmöglichkeit der Erfindung hingewiesen werden. Diese betrifft den Fall der dreidimensionalen FD-OCT-Messung, wenn also alle drei Komponenten des Vektorfeldes D(x,y,z,t) ermittelt werden. Wie bereits besprochen, wird diese Möglichkeit aufgrund der Registrierungsprobleme erfordern, dass man B-Scans vornimmt, um ein vorbestimmtes Schnittvolumen mit Tiefenscans zu durchsetzen. Man verfügt somit über ein vektorwertiges Datenfeld über einem dreidimensionalen Volumen. Dies ermöglicht die Berechnung der Rotation von D in diesem Volumen, also der Wirbelstärke des Verschiebungsfeldes.

Wirbel im Verschiebungsfeld D entsprechen lokalen Verdrehungen des Gewebes, wenn dieses unter Einwirkung der elektromagnetischen Strahlung expandiert. Solche Verdrehungen sind immer dann zu erwarten, wenn das Absorptionsvermögen des Gewebes lokal stark variiert und die Erwärmung benachbarter Bereiche unterschiedlich schnell erfolgt bei ansonsten gleicher Strahlungsleistung. Man wird insbesondere mit Wirbeln parallel zur Gewebeoberfläche rechnen können.

Das Bestimmen von rot D stellt insbesondere für kurzzeitige Strahlungsexpositionen, bei denen Wärmediffusion noch keine Rolle spielt und div D ~ Δt ~ t noch eine gute Näherung ist, eine Möglichkeit dar, das ortsaufgelöste Absorptionsvermögen des Gewebes (das z.B. in der Retina lateral variabel ist) zu untersuchen, wenn man mechanische Parameter wie Elastizitätsmodul oder Schermodul z.B. aus ex vivo Messungen kennt oder übertragen kann. Setzt man hingegen das Absorptionsvermögen als gegeben voraus (beispielsweise aus der weiter oben beschriebenen Beobachtung des Zeitverhaltens der Temperatur), so kann man in vivo Messungen eben dieser mikromechanischen Parameter dort vornehmen, wo starke Unterschiede in der Absorption dies gestatten.

Zusammenfassend lehrt die vorliegende Erfindung, Phaseninformationen eines FD-OCT-Systems aus einem Messgebiet in einem lebenden Gewebe, das während der Messung durch Absorption elektromagnetischer Strahlung lokal erwärmt wird, in lokale und instantane Gewebegeschwindigkeiten zu übersetzen, diese dann zeitlich zu integrieren, um ein Verschiebungsfeld zu berechnen, welches seinerseits danach räumlich zu differenzieren ist, um eine Observable - insbesondere die Gewebeexpansion oder die lokale Gewebeverdrehung - zu erhalten. Die erfindungswesentlichen Schritte sind nur rechnergestützt in der erforderlichen Geschwindigkeit durchführbar und somit notwendig in eine Vorrichtung zu integrieren.

Die Messung der Observablen erfolgt kontaktlos rein optisch. Die Observable ist frei von Translationen durch Eigenbewegungen der lebenden Probe im Messgebiet. Mit den Mitteln der Erfindung kann die Observable ortsaufgelöst und als Funktion der Zeit aufgezeichnet werden. Die Observable besitzt ein physikalisch interpretierbares Zeitverhalten, welches die Anwendung theoretischer Konzepte und Modelle, insbesondere der Wärmeleitungstheorie, erlaubt, um Voraussagen über ihre Zeitentwicklung unter bestimmten Annahmen zu treffen. Das Eintreten dieser Voraussagen kann überwacht werden, und signifikante Abweichungen von den Voraussagen weisen auf eine Verletzung der getroffenen Annahmen hin. Ist dies der Fall, so kann die Bestrahlung des Gewebes deaktiviert werden. Die Dosimetriekontrolle der erfindungsgemäßen Vorrichtung basiert somit auf der Überwachung der Vorhersagbarkeit der durch die Therapiestrahlung verursachten Effekte.

Ist eine Dosimetriekontrolle der zu absorbierenden Strahlung nicht gewünscht oder nicht erforderlich, weil die Strahlungsdosis für Gewebeschäden ohnehin zu gering gewählt wird, so ist besagte Observable überdies geeignet, physikalische Gewebeparameter wie Absorptionsvermögen, Wärmeleitwert oder Schermodul in vivo und in Echtzeit zu ermitteln.

## Patentansprüche

1. Vorrichtung zur Untersuchung oder zur Therapie lebenden Gewebes mittels lokaler Erwärmung des Gewebes durch Absorption elektromagnetischer Strahlung, mit

   - wenigstens einer elektromagnetische Strahlung abgebenden Strahlungsquelle,
   - einer Steuereinheit zur Steuerung der Bestrahlungsparameter der Strahlungsquelle und
   - wenigstens einer FD-OCT-Einrichtung mit einer ein Messlicht abgebenden Lichtquelle zur Beleuchtung desjenigen Gewebebereichs, in dem die elektromagnetische Strahlung vom Gewebe absorbiert wird,

   **gekennzeichnet durch**

eine Recheneinheit zum Ausführen der Schritte:

- Ermitteln der an einem vorbestimmten Messort des Gewebes in Einstrahlrichtung des Messlichts tiefenaufgelösten Gewebegeschwindigkeit aus der Phaseninformation des FD-OCT-Interferenzlichts,
- zeitliches Integrieren der ermittelten Gewebegeschwindigkeit,
- räumliches Differenzieren des berechneten Zeitintegrals, und
- Anzeigen der räumlichen Ableitung als Funktion von Ort und Zeit und/oder Zuführen der räumlichen Ableitung als Funktion von Ort und Zeit zu einem Bewertungsmodul und/oder Zuführen der räumlichen Ableitung als Funktion von Ort und Zeit zur Steuereinheit.

2.  Vorrichtung nach Anspruch 1, **dadurch gekennzeichnet, dass** die Recheneinheit als Zeitintegral ein eindimensionales Verschiebungsfeld des Gewebes und als Ableitung dieses Verschiebungsfeldes nach der Koordinate der Einstrahlrichtung des Messlichts die lineare Gewebeexpansion berechnet.

3.  Vorrichtung nach einem der vorangehenden Ansprüche, **gekennzeichnet durch** eine steuerbare Ablenkvorrichtung für das FD-OCT-Messlicht zum Überstreichen des Messlichtstrahls über jenen Bereich des Gewebes, der den Ort der maximalen Erwärmung **durch** die Strahlungsquelle umgibt.

4.  Vorrichtung nach einem der vorangehenden Ansprüche, **gekennzeichnet durch** optische Mittel zur Einstrahlung des FD-OCT-Messlicht aus wenigstens drei linear unabhängigen Richtungen auf das Gewebe.

5.  Vorrichtung nach Anspruch 4, **dadurch gekennzeichnet, dass** die Recheneinheit als Zeitintegral ein dreidimensionales Verschiebungsfeld des Gewebes und als Divergenz dieses Verschiebungsfeldes die volumetrische Gewebeexpansion berechnet.

6.  Vorrichtung nach Anspruch 4, **dadurch gekennzeichnet, dass** die Recheneinheit als Zeitintegral ein dreidimensionales Verschiebungsfeld des Gewebes und als Rotation dieses Verschiebungsfeldes die lokale Gewebeverdrehung berechnet.

7.  Vorrichtung nach einem der Ansprüche 2 oder 5, **dadurch gekennzeichnet, dass** die Recheneinheit gespeicherte thermische Expansionskoeffizienten des Gewebes enthält und aus der Gewebeexpansion eine Temperaturverteilung berechnet.

8.  Vorrichtung nach Anspruch 7, **dadurch gekennzeichnet, dass** die Recheneinheit gespeicherte Absorptionskoeffizienten und Wärmeleitwerte des Gewebes enthält und aus einer in einem Zeitschritt vorliegenden Temperaturverteilung bei vorgegebenen Bestrahlungsparametern wenigstens die im nächsten Zeitschritt zu erwartende Temperaturverteilung berechnet.

9.  Vorrichtung nach Anspruch 8, **dadurch gekennzeichnet, dass** die Recheneinheit die Steuereinheit der Strahlungsquelle ansteuert und die Änderung der Bestrahlungsparameter in Abhängigkeit von der Abweichung zwischen gemessener und erwarteter Temperaturverteilung veranlasst.

**Claims**

1.  A device for examining or treating living tissue by means of local heating of the tissue by absorbing electromagnetic radiation, comprising:

- at least one electromagnetic radiation source that emits electromagnetic radiation,
- a control unit for controlling the irradiation parameters of the radiation source, and
- at least one FD-OCT apparatus with a light source that delivers a measurement light to light the living tissue in the area of the absorption of the electromagnetic radiation,

**characterized by**
a computational unit that performs the steps of:

- determining, based on the phase information of the FD-OCT interference light, the depth-resolved tissue velocity for a predetermined measuring site on the tissue and along a predetermined irradiation direction of the measurement light,
- integrating the determined tissue velocity with respect to time,
- differentiating the calculated time integral with respect to space, and
- displaying the spatial derivative as a function of space and time and/or providing the spatial derivative as a function of space and time to an evaluation module and/or providing the spatial derivative as a function of space and time to the control unit.

2. The device according to claim 1, **characterized in that** the computational unit calculates a one-dimensional displacement field of the tissue as a time integral, and the linear tissue expansion as a derivative of said displacement field with respect to the coordinate of the irradiation direction of the measurement light.

3. The device according to one of the preceding claims, **characterized by** a controllable deflection unit for the FD-OCT measurement light for sweeping the measurement light beam across that area of the tissue that surrounds the site of maximum heating by the radiation source.

4. The device according to one of the preceding claims, **characterized by** optical means for irradiating the tissue with the FD-OCT measurement light from at least three linear independent directions.

5. The device according to claim 4, **characterized in that** the computational unit calculates a three-dimensional displacement field of the tissue as a time integral, and the volumetric tissue expansion as the divergence of said displacement field.

6. The device according to claim 4, **characterized in that** the computational unit calculates a three-dimensional displacement field of the tissue as a time integral, and the local tissue twisting as the rotation of said displacement field.

7. The device according to one of claims 2 or 5, **characterized in that** the computational unit comprises stored coefficients of thermal expansion of the tissue, and determines a temperature distribution from the tissue expansion.

8. The device according to claim 7, **characterized in that** the computational unit comprises stored absorption coefficients and thermal conductance values of the tissue, and predicts, from a temperature distribution present in a time step and given predetermined irradiation parameters, the temperature distribution to be expected in at least the next time step.

9. The device according to claim 8, **characterized in that** the computational unit controls the control unit of the radiation source and effects the change in the irradiation parameters as a function of the deviation between measured and expected temperature distribution.

**Revendications**

1. Dispositif pour l'examen ou le traitement d'un tissu vivant au moyen de chauffage par absorption d'un rayonnement électromagnétique, avec

    - au moins une source de rayonnement émettant un rayonnement électromagnétique,
    - une unité de commande destinée à contrôler les paramètres d'irradiation de la source de rayonnement et
    - au moins un appareil FD-OCT avec une source lumineuse émettant une lumière de mesure pour éclairer le site tissulaire dans lequel le rayonnement électromagnétique est absorbé par le tissu,

    **caractérisé par**
    une unité de calcul pour l'exécution des étapes suivantes :

    - détermination, en un site prédéterminé de mesure du tissu dans la direction de rayonnement de la lumière de mesure, de la vitesse tissulaire à forte résolution à partir de l'information de phase de la lumière d'interférence FD-OCT,
    - intégration temporelle de la vitesse tissulaire déterminée,
    - différentiation spatiale de l'intégrale temporelle calculée, et
    - affichage de la dérivée spatiale en fonction du site et du temps et/ou introduction de la dérivée spatiale en fonction du site et du temps dans un module d'évaluation et/ou introduction de la dérivée spatiale en fonction du site et du temps dans l'unité de commande.

2. Dispositif selon la revendication 1, **caractérisé en ce que** l'unité de calcul calcule un champ de déplacement tissulaire unidimensionnel en tant qu'intégrale temporelle et l'expansion tissulaire linéaire en tant que dérivée de ce champ de déplacement selon les coordonnées de la direction d'irradiation de la lumière de mesure.

3. Dispositif selon l'une des revendications précédentes, **caractérisé par** un dispositif déflecteur contrôlable pour la lumière de mesure FD-OCT destiné à balayer le faisceau de lumière de mesure sur le dit site tissulaire qui entoure la zone de chauffage maximal par la source de rayonnement.

4. Dispositif selon l'une des revendications précédentes, **caractérisé par** un moyen optique permettant d'irradier le tissu avec la lumière de mesure FD-OCT à partir d'au moins trois directions linéaires indépendantes.

5. Dispositif selon la revendication 4, **caractérisé en ce que** l'unité de calcul calcule un champ de déplacement tissulaire tridimensionnel en tant qu'intégrale temporelle et l'expansion tissulaire volumétrique en tant que divergence de ce champ de déplacement.

6. Dispositif selon la revendication 4, **caractérisé en ce que** l'unité de calcul calcule un champ de déplacement tissulaire tridimensionnel en tant qu'intégrale temporelle et la rotation tissulaire locale en tant que rotation de ce champ de déplacement.

7. Dispositif selon une des revendications 2 ou 5, **caractérisé en ce que** l'unité de calcul contient des coefficients mémorisés d'expansion thermique du tissu et calcule une distribution de la température à partir de cette expansion tissulaire.

8. Dispositif selon la revendication 7, **caractérisé en ce que** l'unité de calcul contient des coefficients mémorisés d'absorption et des valeurs de conductivité thermique du tissu et calcule, à partir de la distribution de température selon des paramètres d'irradiation prédéterminés dans un créneau temporel, au moins la distribution de température à prévoir dans le créneau temporel suivant.

9. Dispositif selon la revendication 8, **caractérisé en ce que** l'unité de calcul contrôle l'unité de commande de la source de rayonnement et provoque la modification des paramètres de rayonnement en fonction de l'écart entre la distribution de températures mesurée et la distribution de températures prévue.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10135944 C2
- WO 0180792 A2
- WO 2007059292 A2
- DE 4309056 A1

**In der Beschreibung aufgeführte Nicht-Patentliteratur**

- **SIGRIST M. W.** Laser Generation of Acoustic Waves in Liquids and Gases. *Journal of Applied Physics,* 1986, vol. 60 (7), R83-R121
- **LIANG et al.** Optical micro-scale mapping of dynamic biomechanical tissue properties. *OPTICS EXPRESS,* 2008, vol. 16 (15), 11052
- **VAKOC et al.** Real-time microscopic visualization of tissue response to laser thermal therapy. *J. Biomed. Opt.,* 2007, vol. 12 (2), 020501-1